# EUROPEAN PATENT APPLICATION

(11) **EP 4 461 322 A1**
(43) Date of publication of application: **13.11.2024**
(21) Application number: 23173185.2
(22) Date of filing: 12.05.2023
(51) Int. Cl.: A61L 24/02, A61L 27/12

(54) **CALCIUM PHOSPHATE POWDER COMPOSITION**

(71) Applicant: Biomimetic Innovations Limited, V14 RW92 Co Clare (IE)
(72) Inventor: PROCTER, Philip, Divonne les Bains, 01220 (FR); ENGQVIST, Håkan, 752 29 Uppsala (SE); PUJARI-PALMER, Michael, 757 57 Uppsala (SE); INSLEY, Gerard, Rathkeale, Co. Limerick (IE)
(74) Representative: Brann AB

(57) **Abstract**

The present invention relates to a powder composition that may be used as an adhesive when mixed with water. The composition is based on a calcium phosphate, a phosphorylated amino acid and an acidic compound.

## Description

### FIELD OF THE INVENTION

The present invention relates to a powder composition of a calcium phosphate compound, a phosphorylated amino acid and an acid compound. Said powder composition may be used as an adhesive. The invention further relates to the use of said powder composition and methods of using the same.

### BACKGROUND

Calcium phosphate (CaP) and in particular hydroxyapatite (Ca₁₀(PO₄)₆(OH)₂, HA), is a mineral that is widely used in medical applications due to its similarity to the mineral components of bone and teeth and its biocompatibility. Furthermore hydroxyapatite is non-toxic, biocompatible and bioactive. This means that hydroxyapatite is not harmful and not recognized as a foreign body and on the other hand that it may have positive effects on remodelling of bone. Hence hydroxyapatite has been widely used in bone repair and as drug/gene delivery vehicle, catalyst, ion adsorption/exchange agent, photoelectric regent and so on. Hydroxy apatite may be formed for example by mixing tetra calcium phosphate (TTCP) and dicalcium phosphate dihydrate (DCPD) at basic conditions.

However, a problem with HA is that it is crystalline and very stable which sometimes makes it challenging for the body to remodel it to incorporate the material into the natural tissue which also takes very long time.

The field of biomaterials includes fixation of implants to tissues as well as tissue repair. The limited mechanical strength of implants in combination adhesives has remained an issue within the field of implants and biomaterials. The repair of soft tissues or internal organs with adhesives has also been broadly unsuccessful.

US2012288446 (US'446) discloses an adhesive comprising a multivalent metal compound, a compound comprising a phosphoserine oligomer or a phosphoserine capped polymer wherein the latter compound is present at 10-90wt%. US'446 discloses experimental data using tetra calcium phosphate (TTCP) as the multivalent metal compound and phosphoserine-ethylene glycol-diglycidyl-phosphoserine for example and obtains adhesive strength of up to 3.76MPa when adhered to bone.

US20130122057 (US'057) discloses a bone restorative composition comprising amino acid phosphate species, a multivalent metal compound and a bioactive glass material containing ionic functional groups. US'057 disclose examples using a composing comprising TTCP as the multivalent metal compound and phosphoserine together with various amounts of Combeite Bioactive glass and water and adhere it to bone. The shear strengths obtained varied between 0.75-2.13MPa.

US8765189 (US'189) teaches an adhesive composition comprising a multivalent metal compound and a phosphoserine like compound in an amount of 10-90wt%. US'189 disclose an adhesion shear strength to cortical bone after 5 minutes of 130-890kPa when using TTCP as the multivalent metal compound and various phosphorylated compounds and 650kPa when using α-TCP and phosphoserine.

Even though there are several tissue adhesives available today on the market none of them are ideal sealants or even adhesives. Cyanoacrylates have shown good adhesion but have shown inflammatory response during degradation. Fibrin glues have low adhesive strength but are more biocompatible. Other adhesives struggle with high costs and long curing times or the lack of tailoring the curing time dependent on the tissue and the situation. Soft tissue adhesive usually contain fibrin or gelatin other various polysaccharides.

Still, general tissue adhesives cannot withstand any major shear forces and none of them have shown or implied that they would work also on soft tissues. There is therefore a need for a composition that may be used as a hard tissue adhesive and as a soft tissue adhesive that provides high shear strength.

### SUMMARY OF THE INVENTION

The aim of the present invention is to overcome the drawbacks of the prior art. Therefore, in a first aspect, the present invention relates to a composition according to claim 1.

In a second aspect the present invention relates to a method of preparing stable amorphous calcium phosphate (ACP) comprising:
a. Preparing a powder composition of a calcium phosphate compound selected from tetra calcium phosphate (TTCP) or tricalcium phosphate (TCP), a phosphorylated amino acid and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof;
b. Adding an aqueous solution to the powder composition to obtain an aqueous composition;
c. Detecting the pH of the aqueous composition; and
d. Optionally adjusting the pH by adding more of the calcium phosphate compound, the phosphorylated amino acid and/or the acidic calcium phosphate in order to obtain a pH equal to or lower than 9.

In a third aspect the present invention relates to an aqueous composition comprising the powder composition according to the present invention and an aqueous solution.

In a fourth aspect the present invention relates to use of the aqueous composition according to the present inventio as a tissue adhesive.

In a fifth aspect the present invention relates to a method of adhering a first tissue to a second tissue using the powder composition according to the present invention comprising:
a. mixing the powder composition according to the present invention with a suitable amount of aqueous solution to obtain a tissue adhesive;
b. applying the tissue adhesive to the first tissue or to the second tissue and optionally leave it for a suitable period of time;
c. bringing the first tissue and the second tissue into contact with each other;
d. optionally applying a pressure on the first and second tissue for a suitable period of time; and
e. letting the tissue adhesive cure.

In a sixth aspect the present invention relates to a kit comprising at least two containers wherein
any one container in the kit can contain any of a calcium phosphate compound selected from tetra calcium phosphate (TTCP) or tricalcium phosphate (TCP) or a combination thereof, a phosphorylated amino acid and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof, and an aqueous solution with proviso that if one container contains the calcium phosphate compound, the phosphorylated amino acid and the acidic compound the aqueous solution has to be in another container.

In a seventh aspect the present invention relates to a composition for use as a tissue adhesive wherein the composition comprises a calcium phosphate compound selected from tetra calcium phosphate (TTCP) or tricalcium phosphate (TCP), a phosphorylated amino acid or phosphocreatine and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof.

### ITEMIZED EMBODIMENTS

In one embodiment of any of the aspects of the present invention the amount of phosphorylated amino acid or phosphocreatine is 10-80mol% on a total dry matter basis, preferably 10-60mol%, more preferably 20-50mol%.
the powder composition comprises a combined amount of the calcium phosphate compound and the acidic compound of 40-90mol%on a total dry matter basis, preferably 50-80mol%.

In one embodiment of any of the aspects of the present invention the mol% ratio between the calcium phosphate compound and the acidic compound of the combined amount is 10-90:90-10.

In one embodiment the amount calcium phosphate compound is preferably 20-50wt% on a dry matter basis, more preferably 25-40wt%, and is preferably TTCP or aTCP.

In one embodiment of any of the aspects of the present invention the calcium phosphate compound is tetra calcium phosphate or α-TCP.

In one embodiment of any of the aspects of the present invention the acid compound is selected from dicalcium phosphate dihydrate (DCPD), dicalcium phosphate anhydrous (DCPA), monocalcium phosphate monohydrate (MCPM), preferably DCPD or DCPA.

In one embodiment of any of the aspects of the present invention the phosphorylated amino acid is phosphoserine, phosphothreonine or phosphotyrosine.

In one embodiment of any of the aspects of the present invention the amount of the acidic compound is 20-50wt% on a total dry matter basis.

In one embodiment of any of the aspects of the present invention the composition comprises 25-60mol%, preferably 30-50mol%, of the phosphorylated amino acid or phosphocreatine, a combined amount of the calcium phosphate compound and the acidic compound of 40-75mol%, preferably 50-70mol%, and wherein the mol% ratio between the calcium phosphate compound and the acidic compound of the combined amount is 60-90:40-10, preferably 65-90:53-10.

In one embodiment of any of the aspects of the present invention the composition comprises 15-40mol%, preferably 20-30mol%, of the phosphorylated amino acid or phosphocreatine, a combined amount of the calcium phosphate compound and the acidic compound of 60-85mol%, preferably 70-80mol%, and wherein the mol% ratio between the calcium phosphate compound and the acidic compound of the combined amount is 10-50:90-50, preferably 10-40:90-60.

In one embodiment of any of the aspects of the present invention the composition further comprises a silicate compound preferably in an amount of 10-30mol% on a total dry matter basis.

In one embodiment of any of the aspects of the present invention the silicate compound is a calcium silicate, preferably mono-, di or tricalcium silicate compound or a mixture of two or more, preferably calcium metasilicate.

In one embodiment of any of the aspects of the present invention the composition is essentially free from water, such as less than 0.5wt%, preferably less than 0.1wt%.

In one embodiment of any of the aspects of the present invention the aqueous composition has a pH equal to or lower than 9, preferably equal to or lower than 8, more preferably equal to or lower than 7, more preferably equal to or lower than 6, more preferably equal to or lower than 5, but preferably not lower than 3.

In one embodiment of any of the aspects of the present invention the pH of the composition is adjusted to be equal to or lower than 8, more preferably equal to or lower than 7, more preferably equal to or lower than 6, more preferably equal to or lower than 5.

In one embodiment of any of the aspects of the present invention the amount of aqueous solution is 15-50wt% on a total weight basis.

In one embodiment of any of the aspects of the present invention at least one of the first and the second tissue is a soft tissue preferably selected from tendon, ligament, fascia, skin, fibrous tissue, muscle, fat, nerve or blood vessel.

In one embodiment of any of the aspects of the present invention at least one of the first and the second tissue is a hard tissue preferably selected from bone or tooth.

In one embodiment of any of the aspects of the present invention the steps are performed in vitro.

All the embodiments presented herein relates to all the aspects of the present invention and may be combined unless stated otherwise.

### BRIEF DESCRIPTION OF THE FIGURES

Figure 1, shear of samples after 3 hours curing at 22°C with different fixed amount of phosphoserine and varying TTCP:DCPD molar ratio.
Figure 2, graph showing the shear value (N) after 16 hours of curing. The samples of 100% TTCP along the y-axis were cured for 24 hours.

### DETAILED DESCRIPTION OF THE INVENTION

In the present application the word "aqueous solution" also encompasses water and water of any purity. The water may be but is not limited to tap water, distilled water or deionized water. The aqueous solution may also be a buffer such as PBS or any suitable saline buffer.

### The powder composition

The composition according to the present invention is a powder composition comprising a calcium phosphate compound selected from tetra calcium phosphate (TTCP), tricalcium phosphate (TCP) or a combination of TTCP and TCP, a phosphorylated amino acid or a phosphocreatine and an acidic compound. The acidic compound is selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof.

The present inventors have found that this powder composition allows when mixed with water the formation and storage of amorphous calcium phosphate (ACP) which when used exhibits surprisingly good mechanical strength and where the composition is easy to handle when adhering tissues or other surfaces together. Especially, the powder composition has shown to be suitable for adhering hard tissue to hard tissue or soft tissue to either hard tissue or a synthetic implant or scaffold but also soft tissue to soft tissue. Further, the composition is also suitable to be used as a general adhesive for adhering to surfaces together.

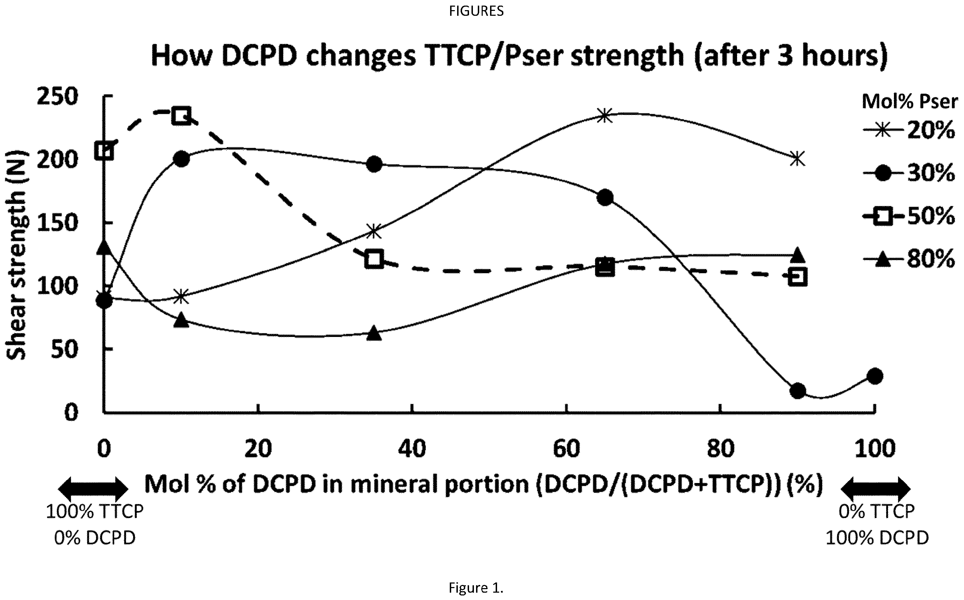

Considering the shear strength shown by TTCP, and that DCPD (an acidic calcium phosphate) shows no shear strength at all on its own, it was surprising to see that the combination of said two components (TTCP and DCPD) in the presence of phosphoserine (a phosphorylated amino acid) resulted in such improved mechanical properties, Figure 1.

What the present inventors found was that when mixing the powder composition with water the obtained aqueous composition not only formed ACP but the ACP also remained stable and did not transform into crystalline hydroxyapatite. Without being bound by theory, the unexpected effect seen is believed to be the result of the combined effects of a lower pH, and the phosphorylated compound (e.g. surface binding of calcium salt particles or grains, ion chelation, increased solubility or dissolution, stabilizing/ destabilizing of nucleation during the cement or crystallization process, etc.) which promoted the formation of ACP and the stabilization of the same. In one embodiment the pH of the aqueous composition is preferably 9 or lower, more preferably 8 or lower, more preferably 7 or lower, more preferably 6 or lower, more preferably 5 or lower. The pH of the composition is determined by creating samples containing 0.15-0.2g of total dry powder, adding liquid at 0.25mL/g, allowing to cure for 5 minutes then submerging the sample into 1mL of water and measuring the pH of the water until it equilibrates to a stable pH reading using any suitable pH measuring device or electrode.

The formed ACP remains stable at room temperature in the aqueous composition, in wet or liquid environments, in at least 1 day, preferably at least 3 days more preferably at least 5 days, more preferably at least 10 days. Stability is determined by measuring x-ray diffraction (XRD) and/or nuclear magnetic resonance (NMR) which both indicate the proportion of solid which is crystalline and non-crystalline (ACP).

The amount of the calcium phosphate compound is preferably 20-50wt% on a dry matter basis, more preferably 25-40wt%. In one preferred embodiment the calcium phosphate is TTCP or α-TCP.

Surprisingly the present inventors found that the improvements of the present composition, with respect to calcium and phosphate salts, were only true for tetracalcium phosphates and tricalcium phosphates. Said calcium phosphates is preferably in form of a powder having a mean particle size of 5-5000nm such as 20nm or larger, 50nm or larger, 100nm or larger, or 300nm or larger, or 500nm or larger, or 800nm or larger, or 3000nm or smaller, or 1500nm or smaller, or 1000nm or smaller. The particles may be spherical or in the shape of flakes.

Phosphorylation is the addition of a phosphate group (PO₄³⁻) to an amino acid or any other molecule. Phosphorylated amino acids according to the present invention may for example be phosphorylated serine, threonine or tyrosine but could also be other amino acids. In one preferred embodiment the phosphorylated amino acid is phosphorylated serine also known as phosphoserine (pSer). The phosphorylated amino acids according to the present invention may be monomers or dimers or trimers.

It is believed that the phosphorylated amino acid or phosphocreatine acts as a curing agent providing improved mechanical strength to the composition. The amount of phosphorylated amino acid or phosphocreatine should be 10-80mol% on a dry matter basis of the powder composition. In order to balance the pH of the aqueous composition when mixing the powder composition with water the amount of phosphorylated amino acid or phosphocreatine may be varied depending on the pH wanted and the amount of the acidic compound. In one embodiment a preferred amount of phosphorylated amino acid or phosphocreatine is 10-60mol% of the solid content such as 12mol% or more, or 15mol% or more, or 18mol% or more, or 35mol% or less, or 32mol% or less, or 30mol% or less, or 28mol% or less, 25mol% or less. In one embodiment the amount of phosphorylated amino acid or phosphocreatine is 20-40mol%, preferably 15-30mol%, on a dry matter basis. An advantage of using said amounts is that the mechanical properties of the obtained adhesive are improved.

The acidic compound is selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof. In a preferred embodiment the acidic compound is DCPD or DCPA, preferably DCPD. These acidic compounds provide sufficient acidity and contribute to the mechanical properties of the adhesive composition when mixed with water.

Acidic compound and calcium phosphate compound is present in the powder composition at a combined amount. In a preferred embodiment said combined amount is 40-90mol% on a dry matter basis, preferably 50-80mol%. Within said ranges a high shear strength was achieved when the powder mixture was mixed with water.

The molar ratio (mol% ratio) between the calcium phosphate compound and the acidic compound is preferably 10-90:90-10. What the inventors saw was that depending on the amount of phosphorylated amino acid or phosphocreatine the shear strength was dependent on said molar ratio. When using mid-range amounts of phosphorylated amino acid or phosphocreatine the amount of calcium phosphate in the combined amount should be higher, while when using low-range amount the amount of calcium phosphate should be lower. In one embodiment the composition comprises 25-60mol%, preferably 30-50mol%, of the phosphorylated amino acid or phosphocreatine, a combined amount of the calcium phosphate compound and the acidic compound of 40-75mol%, preferably 50-70mol%, and wherein the mol% ratio between the calcium phosphate compound and the acidic compound of the combined amount is 60-90:40-10, preferably 65-90:53-10. In another embodiment the composition comprises 15-40mol%, preferably 20-30mol%, of the phosphorylated amino acid or phosphocreatine, a combined amount of the calcium phosphate compound and the acidic compound of 60-85mol%, preferably 70-80mol%, and wherein the mol% ratio between the calcium phosphate compound and the acidic compound of the combined amount is 10-50:90-50, preferably 10-40:90-60.

A preferred powder composition comprises phosphoserine, TTCP and an acidic compound, preferably DCPD, wherein the composition comprises 15-35mol% of phosphoserine, and wherein the molar ratio between the TTCP and the acidic compound is 10-50:90-50, preferably 10-40:90-60.

The powder composition preferably comprises a silicate compound. In one embodiment the composition is essentially free from silicate compounds or totally free from silicate compounds. In another embodiment the amount of silicate compound is 5-30mol%. In one embodiment the amount of silicate is 8mol% or higher, or 12mol% or higher, or 25mol% or less, or 20mol% or less. Silicates further improves the mechanical properties.

The silicate compound may be any suitable compound comprising a silicate i.e. an anionic silicon compound. The silicon compound may be an oxide such as [SiO₄]²⁻ or [Si₂O₇] ⁶⁻ or quartz, feldspars, zeolites, micas, pyroxene etc. In one embodiment the silicate compound is selected from calcium silicate, sodium silicate and aluminum silicate, magnesium silicate, strontium silicate; zirconium silicate; or a mixture of di- and tri-calcium silicate preferably calcium silicate. The silicates may be in the form of a cement such as Portland grey cement or Portland white cement. A mixture of di- and tricalcium silicate may comprise between 0-100wt% of dicalcium silicate and between 0-100wt% of tricalcium silicate such as 30-70wt% of di-calcium silicate and 30-70wt% of tri-calcium silicate. In one embodiment the silicate compound is monocalcium silicate compound, preferably calcium metasilicate

The powder composition is preferably essentially free from water, in other words the amount of water on a total weight basis is preferably less than 0.5wt%, more preferably less than 0.1wt%.

An advantage of the present invention is that the curing time may be tailored so that it cures at the right moment and is dependent on the application. Sometimes the composition should cure very rapidly and sometimes the composition should be mixed or shaped for a while and when applied it might need some adjustment and therefore the curing should be postponed.

A retardant such as sodium citrate may be added to the reaction mixture and the amount may be 0.1-10 wt% (based on solid content). In one embodiment the amount of the retardant is 3.5-7wt% of the solid content. The retardant may be but is not limited to sodium citrate (monosodium, di-sodium or tri-sodium citrate) or citric acid. The composition may further comprise additives such as growth factors, nutrients, anti-oxidants and so on. Still, the composition works without retardants and additives and in one embodiment at least 95wt% of the solid content of the powder composition comprises the calcium phosphate compound, the phosphorylated amino acid or phosphocreatine and the acidic compound, preferably at least 98wt%, more preferably at least 99wt%.

### An aqueous composition

By mixing the powder composition according to the present invention with an aqueous solution an aqueous composition is obtained and can be applied to surfaces for adhesion.

The aqueous solution is preferably water but the aqueous solution may also be a buffer such as PBS or any suitable saline buffer. The water of the aqueous solution may be but is not limited to tap water, distilled water or deionized water.

In order to obtained the wanted properties of the cured composition the pH of the aqueous composition is important. In one preferred embodiment the aqueous composition has a pH equal to or lower than 9, preferably equal to or lower than 8, more preferably equal to or lower than 7, more preferably equal to or lower than 6, more preferably equal to or lower than 5, but preferably not lower than 3.

Based on the total weight of the aqueous composition said composition comprises an aqueous solution in such an amount that the liquid to powder ratio (L/P) is 0.16-0.45 preferably 0.20-0.42 or 0.23-0.42. Powder means the calcium phosphate plus the additive compound and the ratio is a volume (mL) to weight (g) ratio.

### The method of preparing ACP

In order to prepare ACP according to the present invention the powder composition is mixed, in any suitable way, with an aqueous solution preferably water but the aqueous solution may also be a buffer such as PBS or any suitable saline buffer. The water of the aqueous solution may be but is not limited to tap water, distilled water or deionized water.

Amorphous calcium phosphate is formed after mixing the powder composition with the aqueous solution. The amount of aqueous solution added may be 20-50wt% based on the total weight of the obtained aqueous composition. Setting time, flowability or shapeability as well as the mechanical properties are influenced by the amount of aqueous solution added. For example, high amount of water reduces the strength of bonding. The water of the aqueous solution may be distilled or deionized water or any water of high purity but tap water may also be used. The aqueous solution may also be in the form of a buffer or a hydrogel such as hyaluronic acid, polyvinyl alcohol, chitosan, collagen or a combination thereof. By using a hydrogel as the aqueous solution the composition may more easily remain at the wanted location during curing.

### A method of adhering tissues

The aqueous composition according to the present invention may be used for a variety of applications. Due to the ease of applying the aqueous composition and the mechanical strength of the cured composition the composition may be used as a tissue adhesive.

The tissue adhesive is formed by mixing the powder composition according to the present invention with an aqueous solution. Said aqueous solution is preferably water, buffer or a hydrogel. The water of the aqueous solution may be but is not limited to tap water, distilled water or deionized water.

By applying the tissue adhesive to a tissue, hard or soft tissue, another tissue or artificial implant may be adhered and a sufficient mechanical strength is formed between the two surfaces.

Adhering of a first tissue to a second surface may be done by applying the tissue adhesive to the first tissue or to the second surface where the second surface. This may for example be two or more tissues or a tissue to an implant surface or scaffold. The tissue adhesive may also be applied to the second surface as well. The adhesive may be left for a suitable period of time before bringing the two or more tissues or surfaces into contact with each other. The time is dependent on content of the adhesive and the curing time and also on the tissues or materials but non-limiting examples are 10 seconds or longer, or 30 seconds or longer, or 1 minute or longer, or 5 minutes or longer. In one embodiment the composition is left for 20 seconds to 60 seconds before bringing the two or more tissues or surface into contact with each other.

The surfaces are then brought into contact with each other and if necessary pressure may be applied. The pressure is applied depending on the tissue/material and the cure time of the tissue adhesive but non-limiting examples are 10 seconds or longer, or 30 seconds or longer, or 1 minute or longer, or 5 minutes or longer. In one embodiment a pressure is applied for 1-3 minutes. In order to cure the tissue adhesive faster energy may be applied to the composition or to the part of the tissue to which the composition have been applied. This may be done by applying UV, heat or radiation of any suitable type for a couple of seconds up to minutes. The adhesive is then left to cure to the final cured composition. The adhesive may be fully cured after 5 minutes up to 48 hours depending on the composition and the tissue or surface.

The curing time is dependent on the ratios of the inherent components however due to that the tissue adhesive starts to cure when the powder composition is mixed with the aqueous solution the mixing has to be well timed. In certain applications the tissue adhesive should cure rapidly after application of the tissue adhesive and in other applications a slower curing is wanted. The present invention facilitates tailoring of the curing time so that the user may prepare the adhesive on beforehand without having a fully cured adhesive when it is time to apply it or prepare it to obtain a tissue adhesive that is still shapeable or to prepare a tissue adhesive that cures almost instantly.

The method may be performed in vivo or in vitro but some of the steps may be done in vitro followed by steps done in vivo. Injuries that require replacement of large pieces of tissue are unable to heal without intervention. Large pieces of tissue cannot currently be grown for implantation because oxygen and nutrients cannot penetrate deeper than 500um-2mm. One example of a solution to this problem is to grow multiple smaller pieces of tissue and to adhere them together (in vitro or ex vivo), immediately prior to implantation in vivo by using the composition according to the present invention.

The tissue may be a soft tissue and may be selected from but is not limited to tendon, ligament, cartilage, fascia, skin, fibrous tissue, muscle, fat, nerve, blood vessel, liver, stomach, hair, nails, eye lashes, intestines, bladder, brain, eyes, uterus, lungs, esophagus, heart, lung, kidney, spleen and glands. In one embodiment the soft tissue is selected from fascia, skin, fibrous tissue, muscle, fat, nerve, blood vessel, liver, stomach, intestines, bladder, brain, eyes, uterus, lungs, esophagus, heart, lung, kidney, spleen and glands. In one embodiment the soft tissue is cartilage or tendon. In one embodiment the soft tissue adhesive according to the present invention is used to adhere a soft tissue to a hard tissue, or adhere two different soft tissues together. In one embodiment the soft tissue is a tissue having an extra cellular matrix, collagen and elastin. In another embodiment the soft tissue is a tissue having an epithelium. The tissue may also be a hard tissue preferably selected from bone or tooth.

An implant or a scaffold may also be adhered to the tissue by using the composition according to the present invention. The implant or scaffold may be made of synthetic or biological material or a combination thereof. Synthetic materials may be metal, polymers or ceramics where the metals may be titanium, niobium or alloys of the same or aluminum oxide, stainless steel, where the polymers may be polyurethane, polyesters (e.g. polylactic acid, polyglycolic acid, polycaprolactone), polyacrylates (e.g. polymethyl methacrylate, poly(2-hydroxyethyl methacrylate)), polyethers (e.g. polyethylene glycol), polysiloxanes (eg. Silicone), hydrogels (e.g. polyvinyl alcohol) and polyvinyls (e.g. polyethylene, polypropylene, polyisbutylene, polystyrene) and where the ceramics may be calcium phosphates (e.g. hydroxyapatite, monetite, tetra calcium phosphate), metal oxides (e.g. aluminum oxides, zirconium oxides, titanium oxides) or bioglass.

### Kit for preparing the adhesive

A kit comprising the different components of the powder composition may be used to prepare the present composition. The kit comprises at least two containers where the containers may be any suitable type of container such as a bowl, bag, dish, plate, beaker, flask, tin, cup or bottle and may be of any size and shape. Any one container in the kit can contain any of a calcium phosphate compound selected from tetra calcium phosphate (TTCP) or tricalcium phosphate (TCP), a phosphorylated amino acid or phosphocreatine and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof, and an aqueous solution. However if one of the container contains the calcium phosphate compound, the phosphorylated amino acid or phosphocreatine and the acidic compound the aqueous solution has to in another container otherwise the composition cures. In other words one container may comprise the aqueous solution while a second or additional container may comprise the solid components i.e. the calcium phosphate compound, the phosphorylated amino acid or phosphocreatine and the acidic compound. If a silicate compound is used it may be in the form of two or more reactants that may react to form the silicate compound. The two or more reactants may be in the same container or may be in separate compartments. In one embodiment the kit comprises three or more containers. The amount of aqueous solution, the calcium compound, the phosphorylated amino acid or phosphocreatine and the acidic compound in the kit is such that the wanted amounts and ratios of the aqueous composition or tissue adhesive is obtained when mixed.

### EXAMPLES

### Example 1

Materials: Calcium silicate (CS1, calcium metasilicate from Sigma), Tetracalcium phosphate (TTCP, synthesized at Uppsala University), Dicalcium phosphate dihydrate (DCPD, Sigma), alpha tricalcium phosphate (aTCP, Innotere 5µm), and phosphoserine (Flamma) were used as reactants/raw materials.

Samples: Powders were prepared as disclosed in the tables or graphs and were premixed (0.2g total), mixed with water (50uL, 0.25 L/P), and applied to the surface of steel cubes (1cm³), that had been polished with 60 grit silicon carbide polishing paper. Each sample was mixed within 15-20 seconds. Two cubes were pressed together with adhesive between them, using grips (Cocraft grips), and cured at room temperature (22C, 40% relative humidity) for 3 hours, and some samples for 16 hours.

Testing: Samples were tested on an AGS-H, Shimadzu (Shimadzu Europa Gmbh, Duisburg, Germany) mechanical testing machine, using a displacement speed of 1 mm per minute, and a 5kN load cell, in a shear testing setup. Data were analyzed using the manufacturer software, Trapezium-X Lite, version 1.2.0 (Shimadzu Europa). Each group or data point represents the average of N= 2-4 sample size.

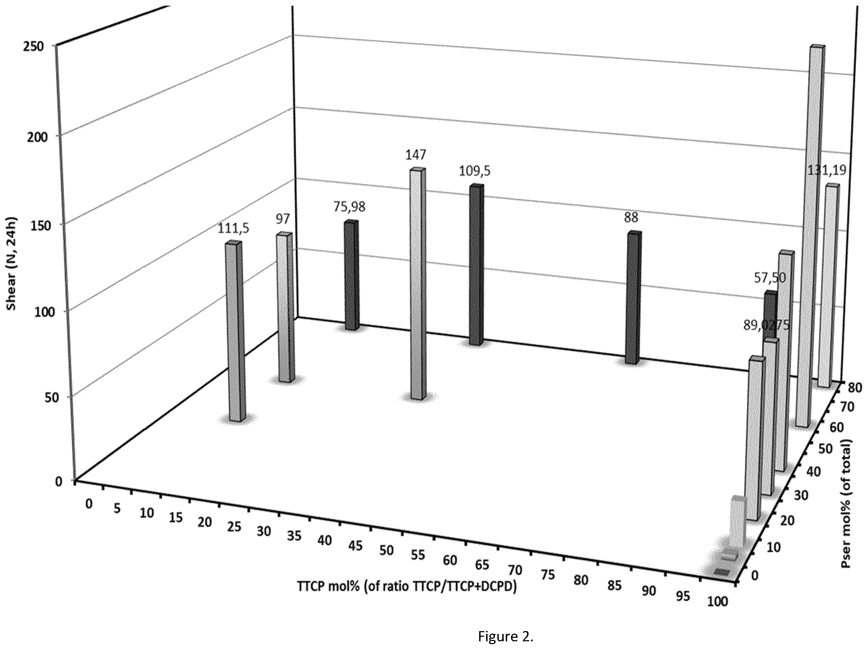

Results: The results are seen in Figure 1. Very high shear strength was seen already after 3 hours of curing. No significant increase in shear strength could be seen (Figure 2) when increasing the curing time to 16 hours showing that the present composition is fully cured within 3 hours.

A few samples comprising monocalcium silicates were prepared and tested as disclosed above. The results are seen in Table 1.

**Table 1. Shear strength after 3 hours of curing of compositions comprising calcium silicate (amounts in mol%).**

| Formulation | Pser | TTCP | DCPD | Cal. Sil. | Set time (min) | Shear (N) | Shear str. (MPa) |
|---|---|---|---|---|---|---|---|
| 25 | 29.2 | 33.7 | 15.8 | 21.4 | 1.3 | 259 | 2.59 |
| 26 | 45.2 | 26.3 | 12.2 | 16.5 | 5.0 | 357 | 3.57 |
| 27 | 21.5 | 373 | 17.5 | 23.7 | 0.8 | 193 | 1.93 |
| 28 | 18.6 | 38.7 | 18.2 | 24.5 | 0.8 | 92 | 0.92 |
| 29 | 67.3 | 15.5 | 7.3 | 9.9 | 4.0 | 147 | 1.47 |

## Claims

1. A powder composition comprising a calcium phosphate compound selected from tetra calcium phosphate (TTCP) or tricalcium phosphate (TCP) or a combination thereof; a phosphorylated amino acid or phosphocreatine, and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof.

2. The powder composition according to claim 1 wherein the amount of phosphorylated amino acid or phosphocreatine is 10-80mol% on a total dry matter basis, preferably 10-60mol%, more preferably 20-50mol%; and wherein the phosphorylated amino acid preferably is phosphoserine, phosphothreonine or phosphotyrosine.

3. The powder composition according to claim 1 or 2 wherein the powder composition comprises a combined amount of the calcium phosphate compound and the acidic compound of 40-90mol%on a total dry matter basis, preferably 50-80mol%.

4. The powder composition according to claim 3 wherein the mol% ratio between the calcium phosphate compound and the acidic compound of the combined amount is 10-90:90-10; wherein the calcium phosphate compound is tetra calcium phosphate or α-TCP, and wherein the amount of the calcium phosphate compound is preferably 20-50wt% on a dry matter basis, more preferably 25-40wt%.

5. The powder composition according to any of the preceding claims wherein the acid compound is selected from dicalcium phosphate dihydrate (DCPD), dicalcium phosphate anhydrous (DCPA), monocalcium phosphate monohydrate (MCPM), preferably DCPD or DCPA; and wherein the amount of the acidic compound preferably is 20-50wt% on a total dry matter basis.

6. The powder composition according to any one of claim 1 to 5 wherein the composition comprises 25-60mol%, preferably 30-50mol%, of the phosphorylated amino acid or phosphocreatine, a combined amount of the calcium phosphate compound and the acidic compound of 40-75mol%, preferably 50-70mol%, and wherein the mol% ratio between the calcium phosphate compound and the acidic compound of the combined amount is 60-90:40-10, preferably 65-90:53-10.

7. The powder composition according to any one of claim 1 to 6 wherein the composition comprises 15-40mol%, preferably 20-30mol%, of the phosphorylated amino acid or phosphocreatine, a combined amount of the calcium phosphate compound and the acidic compound of 60-85mol%, preferably 70-80mol%, and wherein the mol% ratio between the calcium phosphate compound and the acidic compound of the combined amount is 10-50:90-50, preferably 10-40:90-60.

8. The powder composition according to any one of claim 1 to 7 wherein the composition further comprises a silicate compound preferably in an amount of 10-30mol% on a total dry matter basis; wherein the silicate compound preferably is a calcium silicate, preferably mono-, di or tricalcium silicate compound or a mixture of two or more, preferably calcium metasilicate.

9. The powder composition according to any of the preceding claims wherein the composition is essentially free from water, such as less than 0.5wt%, preferably less than 0.1wt%.

10. An aqueous composition comprising the powder composition of any one of claim 1 to 9 and an aqueous solution wherein the aqueous composition preferably has a pH equal to or lower than 9, preferably equal to or lower than 8, more preferably equal to or lower than 7, more preferably equal to or lower than 6, more preferably equal to or lower than 5, but preferably not lower than 3.

11. A method of preparing stable amorphous calcium phosphate (ACP) comprising:
a. Preparing a powder composition of a calcium phosphate compound selected from tetra calcium phosphate (TTCP) or tricalcium phosphate (TCP), a phosphorylated amino acid and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof;
b. Adding an aqueous solution to the powder composition to obtain an aqueous composition;
c. Detecting the pH of the aqueous composition; and
d. Optionally adjusting the pH by adding more of the calcium phosphate compound, the phosphorylated amino acid and/or the acidic calcium phosphate in order to obtain a pH equal to or lower than 9.

12. The method according to claim 11 wherein the pH of the composition is adjusted to be equal to or lower than 8, more preferably equal to or lower than 7, more preferably equal to or lower than 6, more preferably equal to or lower than 5; and wherein the amount of aqueous solution preferably is 15-50wt% on a total weight basis.

13. A method of adhering a first tissue to a second tissue using the powder
composition according to any one of claim 1 to 9 comprising:
a. mixing the powder composition according to claim 1 with a suitable amount of aqueous solution to obtain a tissue adhesive;
b. applying the tissue adhesive to the first tissue or to the second tissue and optionally leave it for a suitable period of time; preferably wherein at least one of the first and the second tissue is a soft tissue preferably selected from tendon, ligament, fascia, skin, fibrous tissue, muscle, fat, nerve or blood vessel, or wherein at least one of the first and the second tissue is a hard tissue preferably selected from bone or tooth;
c. bringing the first tissue and the second tissue into contact with each other;
d. optionally applying a pressure on the first and second tissue for a suitable period of time; and
e. letting the tissue adhesive cure;
wherein the steps are preferably performed in vitro.

14. A kit comprising at least two containers wherein any one container in the kit can contain any of a calcium phosphate compound selected from tetra calcium phosphate (TTCP) or tricalcium phosphate (TCP) or a combination thereof, a phosphorylated amino acid and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof, and an aqueous solution with proviso that if one container contains the calcium phosphate compound, the phosphorylated amino acid and the acidic compound the aqueous solution has to be in another container.

15. A composition for use as a tissue adhesive wherein the composition comprises a calcium phosphate compound selected from tetra calcium phosphate (TTCP) or tricalcium phosphate (TCP) or a combination thereof , a phosphorylated amino acid or phosphocreatine and an acidic compound selected from dicalcium phosphate dihydrate (DCPD), dicalcium phosphate anhydrous (DCPA), monocalcium phosphate monohydrate (MCPM), pyrophosphoric acids or salts thereof and polyphosphitic acids and salts thereof.
